# FASCICULE DE BREVET EUROPEEN

(11) **EP 1 869 042 B1**
(45) Date de publication et mention de la délivrance du brevet: **02.02.2011**
(21) Numéro de dépôt: 06743635.2
(22) Date de dépôt: 05.04.2006
(51) Int. Cl.: C07D 471/04

(54) **NOUVEAUX DERIVES D'ISOINDOLES, COMPOSITIONS LES CONTENANT, LEUR PREPARATION ET LEURS UTILISATIONS PHARMACEUTIQUES NOTAMMENT EN TANT QU'INHIBITEURS D'ACTIVITES DE LA PROTEINE CHAPERONE HSP90**
NEUE ISOINDOLDERIVATE, ZUSAMMENSETZUNGEN DAMIT, IHRE HERSTELLUNG UND PHARMAZEUTISCHE VERWENDUNG, INSBESONDERE ALS HEMMER DER AKTIVITÄT DES CHAPERONPROTEINS HSP90
NOVEL ISOINDOLE DERIVATIVES, COMPOSITIONS CONTAINING SAME, PREPARATION THEREOF AND PHARMACEUTICAL USES THEREOF IN PARTICULAR AS INHIBITORS OF CHAPERONE PROTEIN HSP90 ACTIVITIES

(30) Priorité: 08.04.2005 FR 0503511
(43) Date de publication de la demande: 26.12.2007
(73) Titulaire: Aventis Pharma S.A., 92160 Antony (FR)
(72) Inventeur: MAILLIET, Patrick, F-94120 Fontenay Sous Bois (FR); BERTIN, Luc, F-91560 Crosnes (FR); BENARD, Didier, F-95560 Montsoult (FR); CARREZ, Chantal, F-94320 Thiais (FR); VALLEE, François, F-75013 Paris (FR); BACQUE, Eric, F-91190 Gif sur Yvette (FR)
(74) Mandataire: Le Pennec, Magali
(86) Numéro de dépôt international: PCT/FR2006/000750
(87) Numéro de publication internationale: WO 2006/108948

(56) Documents cités:
- WO-A-2004/056782
- WO-A-2004/072080
- WO-A-2004/096212

## Description

La présente invention concerne de nouveaux composés chimiques qui sont des dérivés isoindoles, ainsi que des compositions qui les contiennent, et leur utilisation comme médicaments.

La présente invention concerne plus particulièrement leurs utilisations pharmaceutiques et notamment leur utilisation en tant qu'inhibiteurs des activités de la protéine chaperone Hsp90.

De tels dérivés selon la présente invention peuvent être notamment des dérivés de 1-(benzimidazol-2-yl)-2,3,4,6-tétrahydro-2H-pyrido[2,1-a]isoindole ou de 1-(azabenzimidazol-2-yl)-2,3,4,6-tétrahydro-2H-pyrido[2,1-a]isoindole ou de 1-(benzimidazol-2-yl)-2,5-dihydro-3H-pyrrolo[2,1-a]isoindole ou de 1-(azabenzimidazol-2-yl)-2,5-dihydro-3H-pyrrolo[2,1-a]isoindole. Plus particulièrement, l'invention concerne, selon un premier aspect, de nouveaux dérivés de benzisoxazole.
Les composés selon la présente invention présentent notamment une activité anticancéreuse, et en particulier une activité inhibitrice de la protéine chaperone Hsp90, et plus particulièrement via l'inhibition de l'activité catalytique de type ATPasique de la protéine chaperone Hsp90.

### Protéines Chaperones

Les chaperones moléculaires de la famille « Heat Schock Proteins » (HSPs), classées en fonction de leur masse moléculaire (Hsp27, Hsp70, Hsp90...), sont des éléments clés de l'équilibre entre la synthèse et la dégradation des protéines cellulaires, responsables du repliement correct des protéines. Elles jouent un rôle vital en réponse au stress cellulaire. Les HSPs, et en particulier Hsp90, sont également impliquées dans la régulation de diverses fonctions majeures de la cellule, via leur association avec diverses protéines clients impliquées dans la prolifération cellulaire ou l'apoptose (Jolly C. et Morimoto R.I., J. N. Cancer Inst.(2000), 92, 1564-72 ; Smith D.F. et al., Pharmacological Rev. (1998), 50, 493-513; Smith D.F., Molecular Chaperones in the Cell, 165-178, Oxford University Press 2001).

Diverses pathologies humaines sont la conséquence d'un repliement incorrect de protéines clés, conduisant notamment à des maladies neurodégénératives suite à l'agrégation de certaines protéines comme dans les maladies d'Alzheimer et de Huntington ou les maladies liées aux prions (Tytell M. et Hooper P.L., Emerging Ther. Targets (2001), 5, 3788-3796). Dans ces pathologies, des approches visant à rompre ou à perturber le fonctionnement des chaperones pourraient être bénéfiques.

### Chaperone Hsp90

La chaperone Hsp90, qui représente 1 à 2 % du contenu protéique de la cellule a été récemment mise en évidence comme une cible particulièrement prometteuse en thérapie anticancéreuse (cf pour revue : Moloney A. et Workman P., Expert Opin. Biol. Ther. (2002), 2(1), 3-24 ; Choisis et al, Drug Discovery Today (2004), 9, 881-888). Cet intérêt tient en particulier aux interactions cytoplasmiques d'Hsp90 avec les principales protéines clients d'Hsp90, protéines qui sont impliquées dans les six mécanismes de progression des tumeurs, tels que définis par Hanahan D. et Weinberg R.A. (Cell (2002), 100, 57-70), à savoir :
- une capacité à proliférer en l'absence de facteurs de croissance: EGFR-R/HER2, Src, Akt, Raf, MEK, Bcr-Abl, Flt-3 ...
- une capacité à échapper à l'apoptose : forme mutée de p53, Akt, survivine ...
- une insensibilité aux signaux d'arrêt de prolifération : Cdk4, Plk , Wee1 ...
- une capacité à activer l'angiogénèse : VEGF-R, FAK, HIF-1, Akt ...
- une capacité à proliférer sans limite réplicative : hTert ...
- une capacité à envahir de nouveaux tissus et à métastaser : c-Met

Parmi les autres protéines clients de Hsp90, des récepteurs aux hormones stéroïdiennes, tels que le récepteur à l'estrogène ou le récepteur à l'androgène, présentent également un intérêt important dans le cadre des thérapies anticancéreuses.

Il a été montré récemment que la forme alpha d'Hsp90 avait également un rôle extracellulaire via son interaction avec la métalloprotéase MMP-2, elle-même impliquée dans l'invasion tumorale (Eustace B.K. et al, Nature Cell Biology (2004), 6, 507-514.

Hsp90 est constituée de deux domaines N- et C-terminaux séparés par une région fortement chargée. L'interaction dynamique entre ces deux domaines, coordonnée par la fixation de nucléotides et de co-chaperones, détermine la conformation de la chaperone et son état d'activation. L'association des protéines clients dépend principalement de la nature des co-chaperones Hsp70/Hsp40, Hop60 etc ... et de la nature du nucléotide ADP ou ATP liée au domaine N-terminal de Hsp 90. Ainsi l'hydrolyse de l'ATP en ADP et le facteur d'échange ADP/ATP contrôlent l'ensemble de la « machinerie » chaperone, et il a été montré qu'il suffit de prévenir l'hydrolyse de l'ATP en ADP - activité ATPasique d'Hsp90 - pour libérer dans le cytoplasme des protéines-clients qui seront alors dégradées au protéasome (Neckers L et Neckers K, Expert Opin. Emerging Drugs (2002), 7, 277-288 ; Neckers L, Current Medicinal Chemistry, (2003), 10, 733-739 ; Piper P.W., Current Opin. Invest. New Drugs (2001), 2, 1606-1610).

### Inhibiteurs d'Hsp90

Les premiers inhibiteurs connus d'Hsp90 sont des composés de la famille des amsamycines, en particulier la Geldanamycine (**1**) et l'Herbimycine A. Des études de rayon X ont montré que la Geldanamycine se lie au site ATP du domaine N-terminal d'Hsp90 où elle inhibe l'activité ATPasique de la chaperone (Prodromou C. et al, Cell (1997), 90, 65-75)

Actuellement le NIH et Kosan BioSciences assurent le développement clinique du 17AAG (**2**), qui est un inhibiteur d'Hsp90 dérivé de la geldanamycine (**1**), qui bloque l'activité ATPasique d'Hsp 90, en se liant au site de reconnaissance de N-terminal de l'ATP. Les résultats des essais cliniques de phase I du 17AAG (**1**) conduisent aujourd'hui à initier des essais de phase II, mais orientent aussi les recherches vers des dérivés plus solubles tel que l'analogue **3** (17DMAG de Kosan BioSciences), porteur d'une chaîne diméthylaminée à la place du résidu méthoxy, et vers des formulations optimisées du 17AAG (CNF1010 de Conforma Therapeutics) :

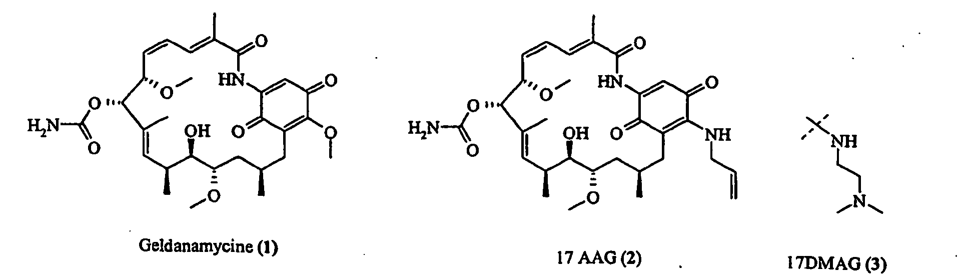

Le radicicol (**4**) est également un inhibiteur d'Hsp 90 d'origine naturelle (Roe S.M. et al, J. Med Chem. (1999), 42, 260-66). Toutefois, si celui-ci est de loin le meilleur inhibiteur *in vitro* d'Hsp90, son instabilité métabolique vis à vis des nucléophiles souffrés le rend difficilement utilisable *in vivo.* Des dérivés oximes bien plus stables tel que le KF 55823 (**5**) ou le KF 25706 ont été développés par la société Kyowa Hakko Kogyo (Soga et al, Cancer Research (1999), 59, 2931-2938)

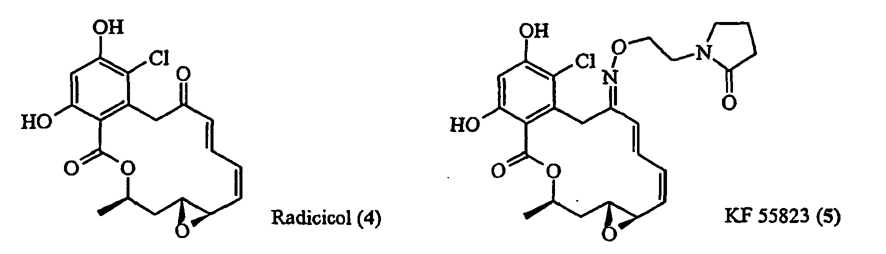

Des structures d'origine naturelle apparentées au radicicol ont également été récemment décrites, comme la zéaralénone (**6**) par la société Conforma Therapeutics (WO 03041643) ou les composés (**7-9**).

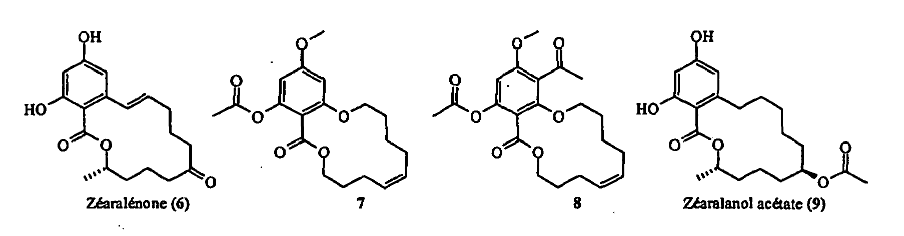

Un inhibiteur d'Hsp90 d'origine naturelle, la novobiocine **(10)** se lie à un site ATP différent situé dans le domaine C-terminal de la protéine (Itoh H. et al, Biochem J. (1999), 343, 697-703.

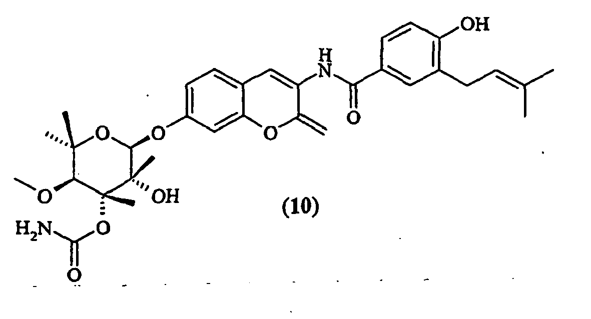

Un depsipeptide, nommé Pipalamycin ou ICI101 vient d'être récemment décrit comme inhibiteur non compétitif du site ATP d'Hsp90 (J. Pharmacol. Exp. Ther. (2004), 310, 1288-1295).

Des purines, comme les composés PU3 (**11**) (Chiosis et al, Chem. Biol. (2001), 8, 289-299) et PU24FCI **(12)** (Chiosis et al, Curr. Canc. Drug Targets (2003), 3, 371-376) ont été également été décrites comme inhibiteurs d'Hsp 90 :

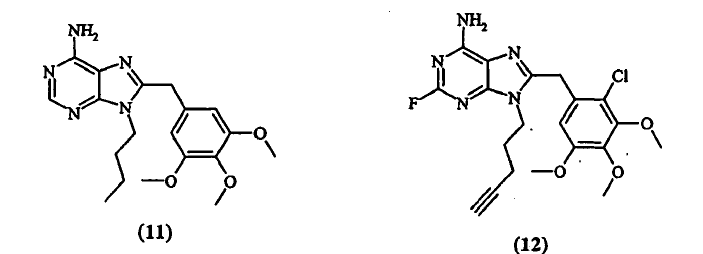

La demande de brevet WO2004/072080 (Cellular Genomics) revendique une famille de 8-hétéroaryl-6-phényl-imidazo[1,2-a]pyrazines comme modulateurs de l'activité d'hsp90.

La demande de brevet WO2004/050087 (Ribotarget/Vernalis) revendique une famille de pyrazoles utiles pour traiter des pathologies liées à l'inhibition des « Heat Shock Proteins » tels que la chaperone Hsp90.

La demande de brevet WO2004/056782 (Vernalis) revendique une nouvelle famille de pyrazoles utiles pour traiter des pathologies liées à l'inhibition des « Heat Shock Proteins » tels que la chaperone Hsp90.

La demande de brevet WO2004/07051 (Vernalis) revendique des dérivés d'arylisoxazoles utiles pour traiter des pathologies liées à l'inhibition des «Heat Shock Proteins » tels que la chaperone Hsp90.

La demande de brevet WO2004/096212 (Vernalis) revendique une troisième famille de pyrazoles utiles pour traiter des pathologies liées à l'inhibition des « Heat Shock Proteins » tels que la chaperone Hsp90.

La demande de brevet WO2005/00300 (Vernalis) revendique de manière plus générale des hétérocycles à 5 chaînons, substitués par des radicaux aryles, utiles pour traiter des pathologies liées à l'inhibition des « Heat Shock Proteins » tels que la chaperone Hsp90.

Enfin la demande de brevet WO2005/00778 (Kyowa Hakko Kogyo) revendique une famille de dérivés de benzophénone comme inhibiteurs d'HsP90, utiles pour le traitement des tumeurs.

La présente invention a ainsi pour objet les produits de formule (I) 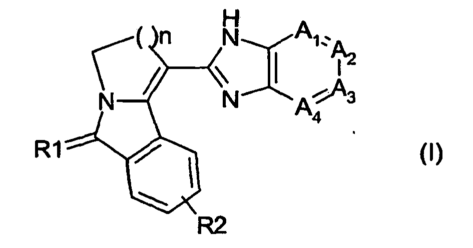 dans laquelle :
A1, A2, A3 et A4, identiques ou différents, représentent CRa ou N ;
n représente l'entier 1 ou 2 ;
R1 représente un atome d'oxygène ou de soufre ou un radical NRb ;
R2 indépendamment sélectionné dans le groupe constitué par H, halogène, CF3, nitro, cyano, méthyle, éthyle, hydroxy, mercapto, amino, méthoxy, thiométhoxy, méthylamino, diméthylamino, acétylamino, carboxy et carboxamido;
Ra est sélectionné dans le groupe constitué par H, halogène, CF3, hydroxy, mercapto, nitro, amino, OR3, SR3, NR3R4, NH-OH, NH-CO-H, NH-CO-OH, NH-CO-NH2, carboxy, cyano, carboxamido, Y-(CH2)p-alkyle, Y-(CH2)p-cycloalkyle, Y-(CH2)p-hétérocycloalkyle, Y-(CH2)p-aryle ou Y-(CH2)p-hétéroaryle, avec Y = O, S, NH, O-C(O), C(O)-NH, NH-C(O), NH-S(O) ou NH-S(O)2, avec p = 1, 2 ou 3, et dans lequel le radical aryle, contient de 6 à 10 chaînons, le radical cycloalkyle contient de 3 à 10 chaînons et le radical hétéroraryle ou hétérocycloalkyle, contient de 4 à 10 chaînons dont 1 à 3 hétéroatomes choisis parmi O, N, ou S ; tous ces radicaux étant éventuellement substitués,
R3 et R4 sont indépendamment choisis parmi l'atome d'hydrogène ou les radicaux alkyles, alkényles, alkynyles, aryles ou hétéroaryle, aralkyle ou hétéroaralkyles; tous les radicaux alkyles, alkènyles, alkynyles, aryles, hétéroaryles, aralkyles ou hétéroaralkyles ci-dessus sont éventuellement substitués ;
Rb est sélectionné dans le groupe constitué par H, (CH2)m-cycloalkyle, (CH2)m-hétérocycloalkyle, (CH2)m-aryle ou (CH2)m-hétéroaryle, avec m = 0, 1, 2, tous ces radicaux étant éventuellement substitués,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

Dans les produits de formule (I) et dans ce qui suit, les termes indiqués ont les significations qui suivent :
- le terme halogène désigne les atomes de fluor, de chlore, de brome ou d'iode et de préférence de chlore ou de brome.
- le terme radical alkyle désigne un radical linéaire ou ramifié renfermant au plus 12 atomes de carbone choisi parmi les radicaux méthyle, éthyle, propyle, isopropyle, butyle, isobutyle, sec-butyle, tert-butyle, pentyle, isopentyle, sec-pentyle, tert-pentyle, néo-pentyle, hexyle, isohexyle, sec-hexyle, tert-hexyle et également heptyle, octyle, nonyle, décyle, undécyle et dodécyle, ainsi que leurs isomères de position linéaires ou ramifiés. On cite plus particulièrement les radicaux alkyle ayant au plus 6 atomes de carbone et notamment les radicaux méthyle, éthyle, propyle, isopropyle, n-butyle, isobutyle, terbutyle, pentyle linéaire ou ramifié, hexyle linéaires ou ramifiés.

- le terme radical alkényle désigne un radical linéaire ou ramifié renfermant au plus 12 atomes de carbone et préférentiellement 4 atomes de carbone choisi par exemple parmi les valeurs suivantes : éthényle ou vinyle, propényl ou allyle, 1-propényle, n-butényle, i-butényle, 3-méthylbut-2-ényle, n-pentényle, hexényle, heptényle, octényle, cyclohexylbutényle et décényle ainsi que leurs isomères de position linéaires ou ramifiés. Parmi les valeurs alkényle, on cite plus particulièrement les valeurs allyle ou butényle.
- le terme radical alkynyle désigne un radical linéaire ou ramifié renfermant au plus 12 atomes de carbone et préférentiellement 4 atomes de carbone choisi par exemple parmi les valeurs suivantes : éthynyle, propynyle ou propargyle, butynyle, n-butynyle, i-butynyle, 3-méthylbut-2-ynyle, pentynyle ou hexynyle ainsi que leurs isomères de position linéaires ou ramifiés. Parmi les valeurs alkynyle, on cite plus particulièrement la valeur propargyle.
- le terme radical alcoxy, que peut représenter par exemple OR3, désigne un radical linéaire ou ramifié renfermant au plus 12 atomes de carbone et préférentiellement 6 atomes de carbone choisi par exemple parmi les radicaux méthoxy, éthoxy, propoxy, isopropoxy, butoxy linéaire, secondaire ou tertiaire, pentoxy, hexoxy et heptoxy ainsi que leurs isomères de position linéaires ou ramifiés,
- le terme alkylthio ou alkyl-S-, que peut représenter par exemple SR3, désigne un radical linéaire ou ramifié renfermant au plus 12 atomes de carbone et représente notamment les radicaux méthylthio, éthylthio, isopropylthio et heptylthio. Dans les radicaux comportant un atome de soufre, l'atome de soufre peut être oxydé en radical SO ou S(O)2.
- le terme radical acyle ou R-CO- désigne un radical linéaire ou ramifié renfermant au plus 12 atomes de carbone dans lequel le radical r représente un atome d'hydrogène, un radical alkyle, cycloalkyle, cycloalkényle, cycloalkyle, hétérocycloalkyle ou aryle, ces radicaux ayant les valeurs indiquées ci-dessus et étant éventuellement substitués comme indiqué : on cite par exemple les radicaux formyle, acétyle, propionyle, butyryle ou benzoyle, ou encore valéryle, hexanoyle, acryloyle, crotonoyle ou carbamoyle
- le terme radical cycloalkyle désigne un radical carbocyclique monocyclique ou bicyclique renfermant de 3 à 10 chaînons et désigne notamment les radicaux cyclopropyle, cyclobutyle, cyclopentyle et cyclohexyle,
- le terme radical cycloalkylalkyle désigne un radical dans lequel cycloalkyle et alkyle sont choisis parmi les valeurs indiquées ci-dessus : ce radical désigne ainsi par exemple les radicaux cyclopropylméthyle, cyclopentylméthyle, cyclohexylméthyle et cycloheptylméthyle.
- Par radical acyloxy, on entend les radicaux acyl-O- dans lesquels acyl a la signification indiquée ci-dessus : on cite par exemple les radicaux acétoxy ou propionyloxy.
- Par radical acylamino, on entend les radicaux acyl-N- dans lesquels acyl a la signification indiquée ci-dessus.
- le terme radical aryle désigne les radicaux insaturés, monocycliques ou constitués de cycles condensés, carbocycliques. Comme exemples de tel radical aryle, on peut citer les radicaux phényle ou naphtyle: on cite plus particulièrement le radical phényle.
- Par arylalkyle on entend les radicaux résultant de la combinaison des radicaux alkyle cités précédemment éventuellement substitués et les radicaux aryles également cités ci-dessus, éventuellement substitués : on cite par exemple les radicaux benzyle, phényléthyle, 2-phénéthyle, triphénylméthyle ou naphthlèneméthyle.
- le terme radical hétérocyclique désigne un radical carbocylique saturé (hétérocycloalkyle) ou insaturé (hétéroaryle) constitué au plus de 6 chaînons interrompus par un ou plusieurs hétéroatomes, identiques ou différents, choisis parmi les atomes d'oxygène, d'azote ou de soufre.

Comme radicaux hétérocycloalkyles, on peut citer notamment les radicaux dioxolane, dioxane, dithiolane, thiooxolane, thiooxane, oxirannyle, oxolannyle, dioxolannyle, pipérazinyle, pipéridinyle, pyrrolidinyle, imidazolidinyle, pyrazolidinyle, morpholinyle ou encore tétrahydrofuryle, tétrahydrothiényle, chromanyle, dihydrobenzofuranyle, indolinyle, pipéridinyle, perhydropyranyle, pyrindolinyle, tétrahydroquinoléinyle, tétrahydroisoquinoléinyle ou thioazolidinyle, tous ces radicaux étant éventuellement substitués.

Parmi les radicaux hétérocycloalkyles, on peut citer notamment les radicaux pipérazinyle éventuellement substitué, pipéridinyle éventuellement substitué, pyrrolidinyle éventuellement substitué, imidazolidinyle, pyrazolidinyle, morpholinyle ou thioazolidinyle.
- Par radical hétérocycloalkylalkyle, on entend les radicaux dans lesquels les restes hétérocycloalkyle et alkyle ont les significations précédentes
- parmi les radicaux hétéroaryles à 5 chaînons on peut citer les radicaux furyle tel que 2-furyle, thiényle tel que 2-thiényle et 3-thiényle, pyrrolyle, diazolyle, thiazolyle, thiadiazolyle, thiatriazolyle, isothiazolyle, oxazolyle oxadiazolyle, 3- ou 4-isoxazolyle, imidazolyle, pyrazolyle, isoxazolyle.

Parmi les radicaux hétéroaryles à 6 chaînons on peut citer notamment les radicaux pyridyle tel que 2-pyridyle, 3-pyridyle et 4-pyridyle, pyrimidyle, pyrimidinyle, pyridazinyle, pyrazinyle et tétrazolyle.
- Comme radicaux hétéroaryles condensés contenant au moins un hétéroatome choisi parmi le soufre, l'azote et l'oxygène, on peut citer par exemple benzothiényle tel que 3-benzothiényle, benzofuryle, benzofurannyle, benzopyrrolyle, benzimidazolyle, benzoxazolyle, thionaphtyle, indolyle, purinyle, quinoléinyle, isoquinoléinyle et naphtyridinyle.

Parmi les radicaux hétéroaryles condensés, on peut citer plus particulièrement les radicaux benzothiényle, benzofurannyle, indolyle ou quinoléinyle, benzimidazolyle, benzothiazolyle, furyle, imidazolyle, indolizinyle, isoxazolyle, isoquinolinyle, isothiazolyle, oxadiazolyle, pyrazinyle, pyridazinyle, pyrazolyle, pyridyle, pyrimidinyle, pyrrolyle, quinazolinyle, 1,3,4-thiadiazolyle, thiazolyle, thiényle et groupes triazolyle, ces radicaux étant éventuellement substitués comme indiqué pour les radicaux hétéroaryles. - le terme amine cyclique, que peut représenter par exemple NR3R4, désigne un radical cycloalkyle renfermant de 3 à 8 chaînons dans lequel un atome de carbone est remplacé par un atome d'azote, le radical cycloalkyle ayant la signification indiquée ci-dessus et pouvant renfermer aussi un ou plusieurs autres hétéroatomes choisi parmi O, S, SO2, N ou NR3 avec R3 tel que défini ci-dessus, comme exemples de telles amines cycliques, on peut citer par exemple les radicaux pyrrolidinyle, pipéridinyle, morpholinyle, pipérazinyle, indolinyle, pyrindolinyle ou tétrahydroquinoléinyle.

Le terme patient désigne les êtres humains mais aussi les autres mammifères. Le terme "Prodrug" désigne un produit qui peut être transformé in vivo par des mécanismes métaboliques (tel que l'hydrolyse) en un produit de formule (I). Par exemple, un ester d'un produit de formule (1) contenant un groupe hydroxyle peut être converti par hydrolyse in vivo en sa molécule mère. Ou encore un ester d'un produit de formule (I) contenant un groupe carboxy peut être converti par hydrolyse in vivo en sa molécule mère.

On peut citer à titre d'exemples des esters de produits de formule (I) contenant un groupe hydroxyle tels que les acétates, citrates, lactates, tartrates, malonates, oxalates, salicylates, propionates, succinates, fumarates, maléates, méthylene-bis-b-hydroxynaphthoates, gentisates, iséthionates, di-p-toluoyl-tartrates, méthanesulfonates, éthanesulfonates, benzenesulfonates, p-toluène-sulfonates, cyclohexylsulfamates et quinates.

Des esters de produits de formule (I) particulièrement utiles contenant un groupe hydroxyle peuvent être préparés à partir de restes acides tels que ceux décrits par Bundgaard et. al., J. Med. Chem., 1989, 32, page 2503-2507 : ces esters incluent notamment des (aminométhyl)-benzoates substitués, dialkylamino-méthylbenzoates dans lesquels les deux groupements alkyle peuvent être liés ensemble ou peuvent être interrompus par un atome d'oxygène ou par un atome d'azote éventuellement substitué soit un atome d'azote alkylé ou encore des morpholino-méthyl)benzoates, e.g. 3- ou 4-(morpholinométhyl)-benzoates, et (4-alkylpiperazin-1-yl) benzoates, e.g. 3- ou 4-(4-alkylpiperazin-1-yl)benzoates.

Le ou les radicaux carboxy des produits de formule (I) peuvent être salifiés ou estérifiés par les groupements divers connus de l'homme du métier parmi lesquels on peut citer, à titre d'exemples non limitatifs, les composés suivants.
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxy-carbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxy-méthyle, propionyloxyméthyle, méthylthiométhyle, diméthyl-aminoéthyle, benzyle ou phénéthyle.

Par carboxy estérifié on entend par exemple les radicaux tels que les radicaux alkyloxycarbonyle par exemple méthoxycarbonyle, éthoxycarbonyle, propoxycarbonyle, butyl ou tert-butyloxycarbonyle, cyclobutyloxycarbonyle, cyclopentyloxycarbonyle ou cyclohexyloxycarbonyle.

On peut également citer des radicaux formés avec les restes esters facilement clivables tels que les radicaux méthoxyméthyle, éthoxyméthyle ; les radicaux acyloxyalkyle tels que pivaloyloxyméthyle, pivaloyloxyéthyle, acétoxyméthyle ou acétoxyéthyle ; les radicaux alkyloxycarbonyloxy alkyle tels que les radicaux méthoxycarbonyloxy méthyle ou éthyle, les radicaux isopropyloxycarbonyloxy méthyle ou éthyle.

Une liste de tels radicaux esters peut être trouvée par exemple dans le brevet européen EP 0 034 536.

Par carboxy amidifié on entend par exemple les radicaux du type -CONR3R4 dans lequel R3 et R4 ont les significations indiquées ci-dessus.

Par radical alkylamino, on entend les radicaux dans lesquels le radical alkyl est choisi parmi les radicaux alkyle cités ci-dessus. On préfère les radicaux alkyle ayant au plus 4 atomes de carbone et on peut citer par exemple les radicaux méthylamino, éthylamino, propylamino ou butylamino, linéaire ou ramifié.

Par radical dialkylamino, on entend les radicaux dans lesquels les radicaux alkyl identiques ou différents sont choisis parmi les radicaux alkyle cités ci-dessus. Comme précédemment on préfère les radicaux alkyle ayant au plus 4 atomes de carbone et on peut citer par exemple les radicaux diméthylamino, diéthylamino, méthyléthylamino linéaire ou ramifié.

Les radicaux NR3R4 peuvent également représenter un hétérocycle qui peut ou non comporter un hétéroatome supplémentaire. On peut citer les radicaux pyrrolyle, imidazolyle, indolyle, pipéridinyle, morpholinyle et pipérazinyle. On préfère les radicaux pipéridinyle, morpholinyle ou pipérazinyle.

Par carboxy salifié on entend les sels formés par exemple avec un équivalent de sodium, de potassium, de lithium, de calcium, de magnésium ou d'ammonium. On peut également citer les sels formés avec les bases organiques telles que la méthylamine, la propylamine, la triméthylamine, la diéthylamine, la triéthylamine. On préfère le sel de sodium.

Lorsque les produits de formule (I) comportent un radical amino salifiable par un acide il est bien entendu que ces sels d'acides font également partie de l'invention. On peut citer les sels fournis avec les acides chlorhydrique ou méthanesulfonique par exemple.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant les mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomère est utilisé dans la présente demande dans son sens le plus large et concerne donc l'ensemble des composés indiqués ci-dessus.

Dans les produits de formule (I) tels que définis ci-dessus et ci-dessous, les radicaux alkyles, alkényles, alkynyles, cycloalkyle, hétérocycloalkyle, aryles, hétéroaryles, aralkyles ou hétéroaralkyles peuvent être éventuellement substitués par un ou plusieurs radicaux, identiques ou différents, choisis parmi les atomes d'halogène ; les radicaux hydroxyle ; cycloalkyle renfermant au plus 6 chaînons ; acyle renfermant au plus 7 atomes de carbone ; cyano ; nitro ; carboxy libre, salifié ou estérifié ; tétrazolyle ; -NH2, -NH(alk), -N(alk)(alk) ; SO2-NH-CO-NH-alkyle ; SO2-NH-CO-NH-phényle; -C(O)-NH2 ; -C(O)-NH(alk) ; -C(O)-N(alk)(alk), -NH-C(O)-(alk), -N(alk)-C(O)-(alk) ; thiényle ; phényle , alkyle, alkylthio, alcoxy et phénoxy eux-mêmes éventuellement substitués par un ou plusieurs radicaux choisis parmi les atomes d'halogène et les radicaux hydroxyle, alcoxy, alkyle, -NH2, -NH(alk) et -N(alk)(alk). Plus particulièrement, dans les produits de formule (I) tels que définis ci-dessus et ci-dessous, les radicaux alkyles, alkényles, alkynyles, cycloalkyle, hétérocycloalkyle, aryles, hétéroaryles, aralkyles ou hétéroaralkyles peuvent être éventuellement substitués par un ou plusieurs radicaux, identiques ou différents, choisis parmi les atomes d'halogène ; les radicaux hydroxyle ; carboxy libre, salifié ou estérifié; -NH2, -NH(alk), -N(alk)(alk) ; phényle, alkyle et alcoxy eux-mêmes éventuellement substitués par un ou plusieurs radicaux choisis parmi les atomes d'halogène et les radicaux hydroxyle, alcoxy, alkyle, -NH2, -NH(alk) et -N(alk)(alk).
Encore plus particulièrement, dans les produits de formule (I) tels que définis ci-dessus et ci-dessous, les radicaux alkyles, alkényles, alkynyles, cycloalkyle, hétérocycloalkyle, aryles, hétéroaryles, aralkyles ou hétéroaralkyles peuvent être éventuellement substitués par un ou plusieurs radicaux, identiques ou différents, choisis parmi les atomes d'halogène et les radicaux hydroxyle et alcoxy.

La présente invention concerne ainsi notamment les produits de formule (I) tels que définis ci-dessus dans laquelle
A1, A2, A3 et A4, identiques ou différents, sont tels que A1 et A4 identiques ou différents représentent CRa et A2 et A3 représentent N ou CRa,
les substituants n; Ra, R1 et R2 ayant les significations indiquées ci-dessus;
lesdits produits de formule (1) étant sous toutes les formes isomères possibles racémiques, énantiomères et diastéréo-isomères, ainsi que les sels d'addition avec les acides minéraux et organiques ou avec les bases minérales et organiques desdits produits de formule (I).

La présente invention concerne ainsi notamment les produits de formule (I) tels que définis ci-dessus dans laquelle
A1, A2, A3 et A4, identiques ou différents, sont tels que A1 et A4 identiques ou différents représentent CRa et A2 et A3 représentent N ou CRa, avec Ra représente H ou OH,
les substituants n; R1 et R2 ayant les significations indiquées ci-dessus;
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

La présente invention concerne ainsi notamment les produits de formule (I) tels que définis ci-dessus dans laquelle
A1, A2, A3 et A4 sont tels que A1 et A4 représentent CH et A2 et A3 identiques ou différents représentent N, CH ou COH,
les substituants n; R1 et R2 ayant les significations indiquées ci-dessus;
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

La présente invention concerne ainsi notamment les produits de formule (I) tels que définis ci-dessus dans laquelle
R1 représente un atome d'oxygène ou un radical NRb avec Rb représente H ou (CH2)m-hétéroaryle, avec m = 0,1, 2,
les substituants A1, A2, A3, A4, n et R2 ayant les significations indiquées ci-dessus;
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

Lorsque Rb représente (CH2)m-hétéroaryle, la présente invention concerne notamment les valeurs de Rb dans laquelle m représente 1 et hétéroaryle représente un radical pyridyle éventuellement substitué par un radical amino NR3R4 avec R3 et R4 tels que définis ci-dessus, les autres substituants A1, A2, A3, A4, n et R2; ayant les significations indiquées ci-dessus;
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

La présente invention concerne ainsi notamment les produits de formule (I) tels que définis ci-dessus dans laquelle n représente 2,
les substituants A1, A2, A3, A4; R1 et R2 ayant les significations indiquées ci-dessus ;
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

La présente invention concerne particulièrement les produits de formule (I) tels que définis ci-dessus dans laquelle
A1, A2 et A4 représentent CH et A3 représentent N, CH ou COH,
n représente l'entier 2 ;
R1 représente un atome d'oxygène ;
R2 représente H,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

La présente invention concerne tout particulièrement les produits de formule (I) tels que définis ci-dessus dont les noms suivent :
- le 1-{3-H-imidazo[4,5-c]pyridin-2-yl}-3,4-dihydro-2H-pyrido[2,1-a] isoindole-6-one,
- le 1-{1-H-6-hydroxy-benzimidazol-2-yl}-3,4-dihydro-2H-pyrido[2,1-a] isoindole-6-one,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

La présente invention a également pour objet des procédés de préparation des produits de formule (I) selon la présente invention.

La synthèse du noyau 2,3,4,6-tétrahydro-2H-pyrido[2,1-a]isoindole est peu décrite dans la littérature

Quelques dérivés substitués en position 1 de ce noyau, comme ceux présentés ci-dessous sont connus :

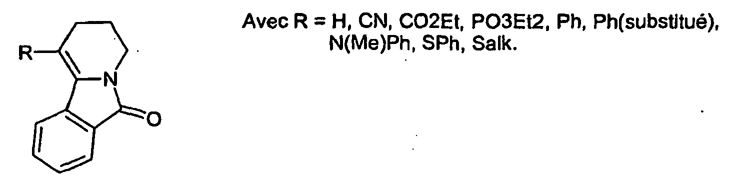

Gourves J.P. et al (Eur. J. Org. Chem. 1999, 3489) décrivent une synthèse mettant en oeuvre une cyclisation intramoléculaire par réaction de Horner-Wadworth-Emmons comme étape clé :

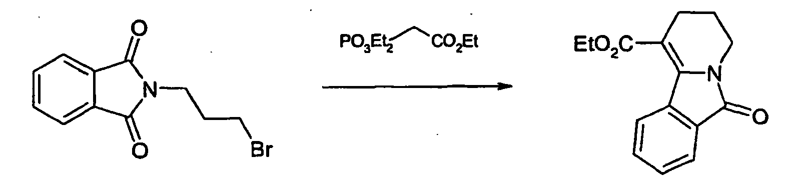

Le brevet JP 49102699 décrit la préparation du 1-phenylthio- et du 1-*tert*butylthio-6-oxo-2,3,4,6-tétrahydropyrido[2,1-a]isoindole, de manière assez similaire mais en mettant en jeu une étape de cyclisation induite par irradiation photochimique des N-(4-phényl- ou 4-*tert*butylthio-butyl)phtalimides :

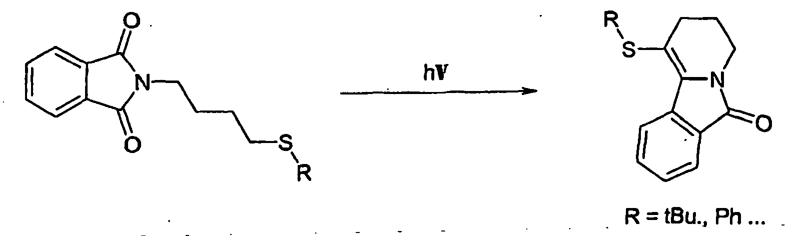

Deok-Chan et al (Tetrahedron Lett. 1996, 37, 2577-80) décrivent la synthèse du noyau 6-oxo-2,3,4,6-tétrahydropyrido[2,1-a]isoindole par cyclisation réductrice du N-(4-iodo-butylthio-butyl)phtalimide induite par de l'iodure de samarium :

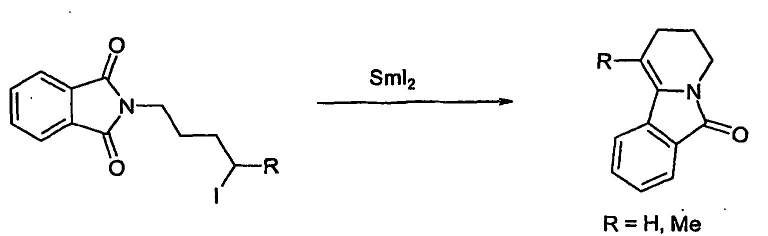

Earl R.A. et Volhardt P.C. (Heterocycles, 1982, 19, 265-71) décrivent la synthèse du noyau 6-oxo-2,3,4,6-tétrahydropyrido[2,1-a]isoindole par une réaction de rétro-ène imino Diels-Alder :

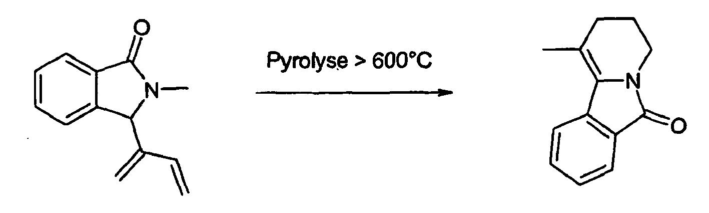

Mazzochi et al (Tetrahedron Lett. 1983, 24, 143-46) décrivent la synthèse du noyau 6-oxo-2,3,4,6-tétrahydropyrido[2,1-a]isoindole par une réaction intramoléculaire de Paterno-Buchi :

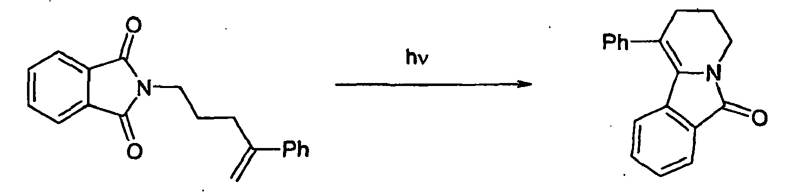

Enfin Malacria M. et al (Organic Lett. 2003, 5, 5095-97) décrivent la synthèse du noyau 6-oxo-2,3,4,6-tétrahydropyrido[2,1-a]isoindole par une réaction de cyclisation radicalaire tandem :

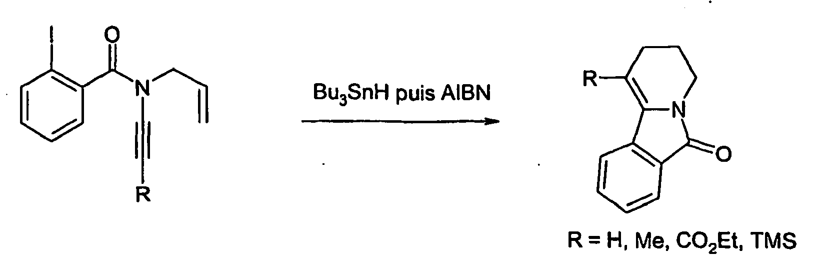

Le squelette 2,3,4,6-tétrahydro-2H-pyrido[2,1-a]isoindole est également parfois retrouvé dans des structures tétracycliques plus complexes tels que les dérivés de la mérocyanine décrits, dans le brevet IT671447, les dérivés d'hémiporphyrazine, décrits dans le brevet SU 178001, ou les colorants utilisés comme copolymères dans l'élaboration de fibres textiles synthétiques, tels que ceux décrits dans les brevets DE 2128326, BE662237, US3221041 ou NL 6504566.

La synthèse du noyau 2,5-dihydro-3H-pyrrolo[2,1-a]isoindole est peu décrite dans la littérature

Quelques dérivés substitués en position 1 de ce noyau, comme ceux présentés ci-dessous sont connus :

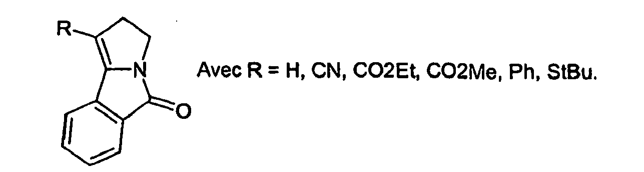

Petter R.C. et al (J. Org. Chem. 1990, 55, 3088-3097) décrivent une synthèse mettant en oeuvre une cyclisation intramoléculaire par réaction de Horner-Wadworth-Emmons comme étape clé :

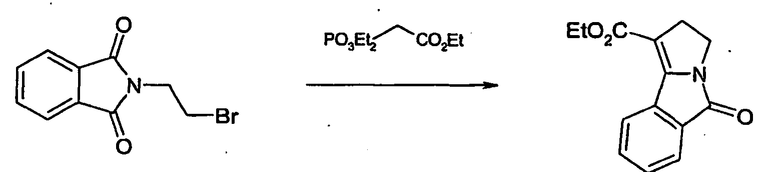

Muchovski et al (Tetrahedron Lett. 1980, 21, 4585-88) décrivent une synthèse par ouverture d'un sel de triphénylphosphonium dun ester cyclopropanoique par un phtalimie :

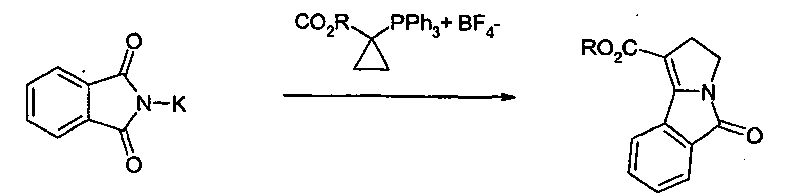

Deok-Chan et al (Tetrahedron Lett. 1996, 37, 2577-80) décrivent une synthèse par cyclisation réductrice du N-(4-iodo-butylthio-butyl)phtalimide induite par de l'iodure de samarium :

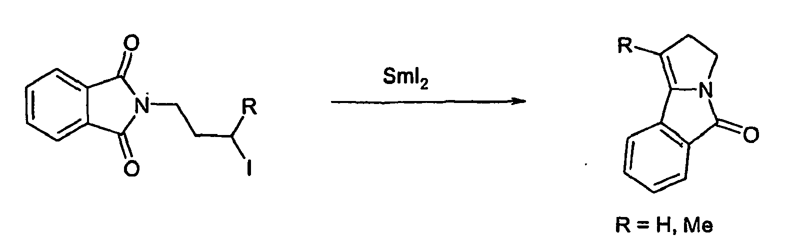

Yoon et al (J. Amer. Chem. Soc. 1995, 117, 2698-2710) décrivent une synthèse par cycladdition d'un ylure d'azométhine avec un acrylate suivie de la déshydratation de l'adduit en milieu acide :

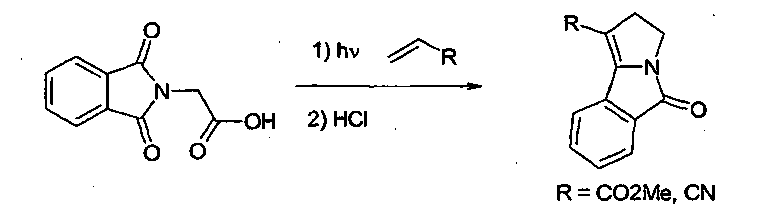

Sato et al (Liebigs Ann. Chem. 1985, 1099-1108) décrivent une synthèse mettant en jeu une étape de cyclisation induite par irradiation photochimique des N-(4-phényl- ou 4-*ter*tbutylthio-butyl)phtalimides :

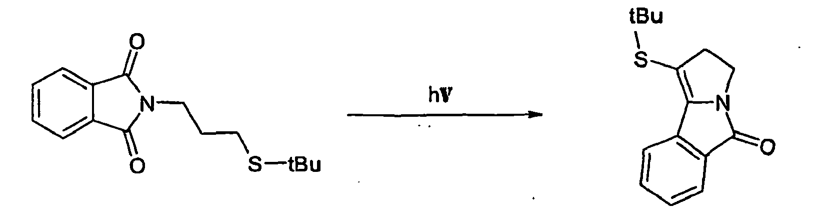

De plus le squelette 2,5-dihydro-3H-pyrrolo[2,1-a]isoindole est retrouvé dans de nombreux composés tétracycliques.

### Méthodes générales de synthèse

Une première méthode générale originale, décrite dans le schéma général 1, s'inspirant des travaux ci-dessus, a été développée et s'est avérée particulièrement avantageuse dans le cadre de la présente invention, en particulier pour la synthèse de dérivés de type 6-oxo-2,3,4,6-tétrahydropyrido[2,1-a]isoindole :

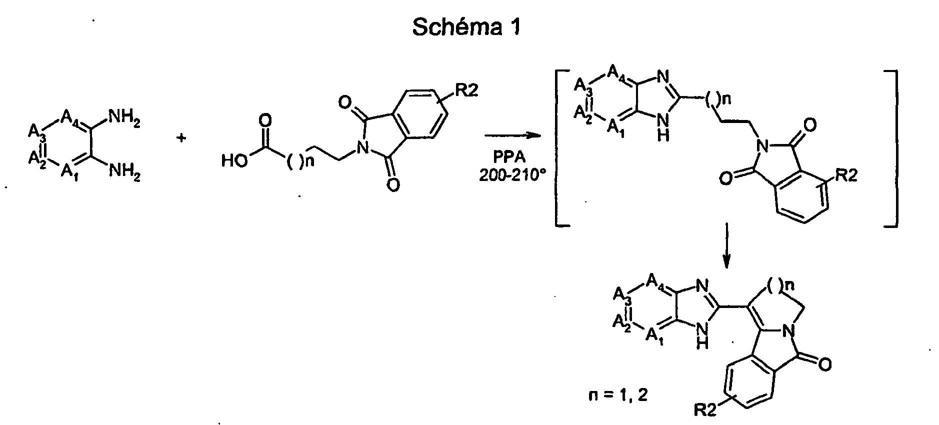

La transformation du radical =O en radicaux =R1 tels que définis dans la formule générale (I) peut être effectuée selon les méthodes générales connues de l'homme de l'art, en particulier celles décrites dans :
- Comprehensive Organic Chemistry, par D. Barton et al. (Pergamon Press) ;
- Advanced Organic Chemistry, par J. Marsh (Wiley Interscience).

Une seconde méthode générale de synthèse a été développée à partir de l'ester méthylique de l'acide 6-oxo-2,3,4,6-tétrahydro-1H-pyrido[2,1-a]isoindole-1-carboxylique ou de l'ester méthylique de l'acide 6-oxo-2,5-dihydro-3H-pyrrolo[2,1-a]isoindole-1-carboxylique, par formation du noyau de type benzimidazole. Il a été trouvé particulièrement avantageux, dans le cadre de la présente invention, d'opérer en deux étapes successives selon le schéma 2 :

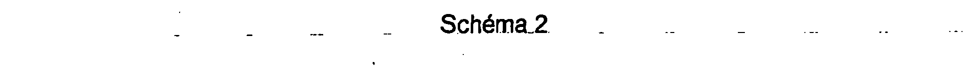

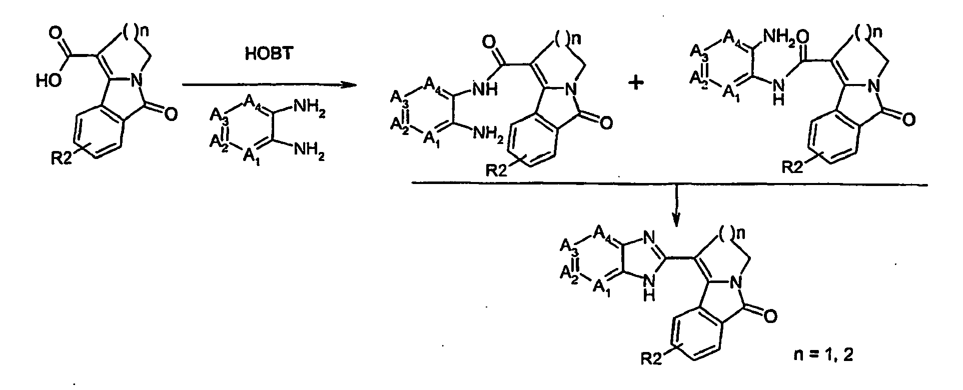

Les produits objet de la présente invention sont doués de propriétés pharmacologiques intéressantes : on a constaté qu'ils possédaient notamment des propriétés inhibitrices de l'activité ATPasique de protéines chaperones. Parmi ces protéines, on cite notamment HSP90.

L'invention a donc pour objet l'application, à titre de médicaments, des produits de formule générale (I) pharmaceutiquement acceptables.

L'invention a plus particulièrement pour objet l'application à titre de médicaments, des produits dont les noms suivent :
- le 1-{3-H-imidazo[4,5-c]pyridin-2-yl}-3,4-dihydro-2H-pyrido[2,1-a]isoindole-6-one,
- le 1-{1-H-6-hydroxy-benzimidazol-2-yl}-3,4-dihydro-2H-pyrido[2,1-a]isoindole-6-one,
ainsi que leurs prodrugs, lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

Les produits peuvent être administrés par voie parentérale, buccale, perlinguale, rectale ou topique.

L'invention a aussi pour objet les compositions pharmaceutiques, caractérisées en ce qu'elles renferment, à titre de principe actif, un au moins des médicaments de formule générale (I).

L'invention a ainsi pour objet les compositions pharmaceutiques telles que définies ci-dessus caractérisées en ce qu'elles sont utilisées comme médicaments, en particulier pour la chimiothérapie de cancers. L'invention a ainsi pour objet les compositions pharmaceutiques telles que définies ci-dessus contenant en plus, des principes actifs d'autres médicaments de chimiothérapie contre le cancer.

Ces compositions peuvent être présentées sous forme de solutions ou de suspensions injectables, de comprimés, de comprimés enrobés, de capsules, de sirops, de suppositoires, de crèmes, de pommades et de lotions. Ces formes pharmaceutiques sont préparées selon les méthodes usuelles. Le principe actif peut être incorporé à des excipients habituellement employés dans ces compositions, tels que les véhicules aqueux ou non, le talc, la gomme arabique, le lactose, l'amidon, le stéarate de magnésium, le beurre de cacao, les corps gras d'origine animale ou végétale, les dérivés paraffiniques, les glycols, les divers agents mouillants, dispersants ou émulsifiants, les conservateurs.

La dose usuelle, variable selon le sujet traité et l'affection en cause, peut être, par exemple, de 10 mg à 500 mg par jour chez l'homme, par voie orale.

Les produits répondant à la formule générale (I) tels que définis ci-dessus présentent ainsi une activité inhibitrice de la chaperone Hsp90 importante.

La présente invention a ainsi également pour objet l'utilisation de produits de formule (I) telle que définie ci-dessus ou de sels pharmaceutiquement acceptables desdits produits de formule (I) pour la préparation d'un médicament destiné à prévenir ou traiter une maladie caractérisée par le dérèglement de l'activité de la protéine HSP90.

La présente invention a ainsi pour objet l'utilisation de produits de formule (I) tels que définis ci-dessus ou de sels pharmaceutiquement acceptables desdits produits de formule (I) pour la préparation de médicaments destinés à inhiber l'activité de la protéine HSP90.

La présente invention a ainsi pour objet l'utilisation de produits de formule (I) tels que définis ci-dessus ou de sels pharmaceutiquement acceptables desdits produits de formule (I) dans laquelle la maladie à prévenir ou traiter est chez un mammifère.

Des tests donnés dans la partie expérimentale ci-après illustrent l'activité inhibitrice de produits de la présente invention vis-à-vis de telles protéines.

La présente invention a ainsi également pour objet l'utilisation de produits de formule (I) tels que définis ci-dessus ou de sels pharmaceutiquement acceptables desdits produits de formule (I) pour la préparation d'un médicament destiné à traiter des cancers.

La présente invention a ainsi également pour objet l'utilisation de produits de formule (I) tels que définis ci-dessus ou de sels pharmaceutiquement acceptables desdits produits de formule (I) dans laquelle la maladie à traiter est un cancer de tumeurs solides ou liquides.

Les propriétés des produits de formule générale (I) de la présente invention les rendent donc utilisables comme médicaments notamment pour le traitement de tumeurs malignes.

Parmi ces cancers, la présente invention s'intéresse tout particulièrement au traitement des tumeurs solides et au traitement de cancers résistants aux agents cytotoxiques

La présente invention a ainsi également pour objet l'utilisation de produits de formule (I) tels que définis ci-dessus ou de sels pharmaceutiquement acceptables desdits produits de formule (I) dans laquelle la maladie à traiter est un cancer résistant aux agents cytotoxiques.

La présente invention a ainsi également pour objet l'utilisation de produits de formule (I) telle que définie tels que définis ci-dessus ou de sels pharmaceutiquement acceptables desdits produits de formulé (I) pour la préparation d'un médicament destiné à traiter des cancers parmi lesquels les cancers du poumon, du sein et de l'ovaire, les glioblastomes, les leucémies myéloïdes chroniques, les leucémies lymphoblastiques aigües, les cancers de la prostate, du pancréas et du colon, les mélanomes métastatiques, les tumeurs de la thyroïde et les carcinomes rénaux.

Aussi parmi les principales indications potentielles d'inhibiteurs d'Hsp90, on peut citer, à titre non limitatif :
les cancers du poumon « à non petites cellules », les cancers du sein, les cancers de l'ovaire et les glioblastomes qui surexpriment EGF-R ou HER2 ;
les leucémies myéloïdes chroniques qui surexpriment Bcr-Abl ;
les leucémies lymphoblastiques aigues qui surexpriment Flt-3 ;
les cancers du sein, de la prostate, du poumon, du pancréas, du colon ou de l'ovaire qui surexpriment Akt ;
les mélanomes métastatiques et les tumeurs de la thyroïde qui surexpriment la forme mutée de la protéine B-Raf ;
les cancers de la prostate androgène-dépendants et androgène-indépendants ;
les cancers du sein estrogène-dépendants et estrogène-indépendants ;
les carcinomes rénaux qui surexpriment HIF-1a ou la protéine c-met mutée ...

La présente invention concerne aussi l'utilisation de produits de formule (I) telle que définie ci-dessus ou de sels pharmaceutiquement acceptables desdits produits de formule (I) pour la préparation d'un médicament destiné à la chimiothérapie de cancers.

A titre de médicaments selon la présente invention destinés à la chimiothérapie de cancers, les produits de formule (I) selon la présente invention peuvent être utilisés seuls ou en association avec chimiothérapie ou radiothérapie ou alternativement en association avec d'autres agents thérapeutiques.

La présente invention a ainsi également pour objet l'utilisation de produits de formule (I) tels que définis ci-dessus ou de sels pharmaceutiquement acceptables desdits produits de formule (I) pour la préparation de médicaments destinés à la chimiothérapie de cancers utilisés seuls ou en association.

La présente invention concerne ainsi notamment les compositions pharmaceutiques telles que définies ci-dessus contenant en plus, des principes actifs d'autres médicaments de chimiothérapie contre le cancer.

De tels agents thérapeutiques peuvent être des agents anti-tumoraux utilisés communément.

La présente invention a ainsi également pour objet l'utilisation de produits de formule (I) tels que définis ci-dessus ou de sels pharmaceutiquement acceptables desdits produits de formule (I) pour la préparation de médicaments destinés à être utilisés seuls ou en association avec chimiothérapie ou radiothérapie ou alternativement en association avec d'autres agents thérapeutiques.

La présente invention a ainsi également pour objet l'utilisation de produits de formule (I) tels que définis ci-dessus ou de sels pharmaceutiquement acceptables desdits produits de formule (I) dans laquelle les agents thérapeutiques peuvent être des agents anti-tumoraux utilisés communément. Comme exemples d'inhibiteurs connus de protéines kinases, on peut citer notamment la butyrolactone, le flavopiridol, la 2-(2-hydroxyéthylamino)-6-benzylamino-9-méthylpurine, l'olomucine, le Glivec ainsi que l'Iressa.

Les produits de formule (I) selon la présente invention peuvent ainsi également être avantageusement utilisés en combinaison avec des agents anti-prolifératifs : à titre d'exemples de tels agents anti-prolifératifs mais sans toutefois se limiter à cette liste, on peut citer les inhibiteurs d'aromatase, les antiestrogènes, les inhibiteurs de topoisomérase I, les inhibiteurs de topoisomérase II, les agents actifs sur les microtubules, les agents d'alkylation, les inhibiteurs d'histone désacétylase, les inhibiteurs de farnésyl transférase, les inhibiteurs de COX-2, les inhibiteurs de MMP, les inhibiteurs de mTOR, les antimétabolites antinéoplastique, les composés du platine, les composés faisant décroître l'activité des protéines kinases et également les composés anti-angiogéniques, les agonistes de la gonadoréline, les anti-androgènes, les bengamides, les biphophonates et le trastuzumab.

On peut citer ainsi à titre d'exemples, des agents anti-microtubules comme les taxoides, vinka-alkaloides, des agents d'alkylation tels que cyclophosphamide, des agents DNA-intercalant comme le cis-platinum, des agents interactifs sur topoisomérase comme la camptothécine et dérivés, les anthracyclines comme l'adriamycine, des antimétabolites comme le 5-fluorouracile et dérivés et analogues.

La présente invention concerne donc des produits de formule (I) comme inhibiteurs de protéines kinases, lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I) ainsi que leurs prodrugs.

La présente invention concerne particulièrement des produits de formule (I) tels que définis ci-dessus comme inhibiteurs de HSP90, lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

Les produits de formule (I) selon la présente invention peuvent être préparés par l'application ou l'adaptation de méthodes connues et notamment des méthodes décrites dans la littérature comme par exemple celles décrites par R.C.Larock dans: Comprehensive Organic Transformations, VCH publishers, 1989.

Dans les réactions décrites ci-après, il peut être nécessaire de protéger des groupes fonctionnels réactifs tels que par exemple des groupements hydroxy, amino, imino, thio ou carboxy, quand ceux-ci sont désirés dans le produit final mais quand leur participation n'est pas souhaitée dans les réactions de synthèse des produits de formule (I). On peut utiliser des groupes protecteurs conventionnels en accord avec les pratiques usuelles standard comme ceux décrits par exemple par T.W. Greene and P.G.M.Wuts dans "Protective Groups in Organic Chemistry" John Wiley and Sons, 1991.

Les exemples dont la préparation suit illustrent la présente invention sans toutefois la limiter.

### Exemples illustrant l'invention

### Exemple 1 : 1-{3-H-imidazo[4,5-c]pyridin-2-yl}-3,4-dihydro-2H-pyrido[2,1-a] isoindole-6-one

Dans un ballon de 250 ml, on introduit 4 g de pyridine-3,4-diamine et 9.5 g d'acide 5-(1,3-dioxo-1,3-dihydro-isoindol-2-yl)pentanoïque puis 45 g d'acide polyphosphorique (PPA). Le mélange solide est chauffé à 210°C au bain d'huile. Lors de la réaction, on note la formation transitoire de 2-{4-(3H-imidazo[4,5-c] pyridin-2-yl)butyl}isoindole-1,3-dione. Après 16 heures de chauffage, la réaction est totale. Après refroidissement le mélange réactionnel est repris dans l'eau. Les impuretés sont. éliminées par extraction à l'acétate d'éthyle. La phase aqueuse est neutralisée (pH7) à la soude 2N. Le produit est récupéré après 6 extractions successives par un mélange d'acétate d'éthyle et de méthanol (9/1 en volumes). Après purification par flash-chromatographie sur silice, en éluant avec un mélange de dichlorométhane et de méthanol (95/5 en volumes), on obtient 1.46 g de 1-{3-H-imidazo[4,5-c]pyridin-2-yl}-3,4-dihydro-2H-pyrido[2,1-a] isoindole-6-one, sous forme d'un solide blanc dont les caractéristiques sont les suivantes :
- Spectre de RMN 1H (400 MHz DMSO-d6) : 2,08 (m, 2H); 2,88 (t, J = 6,0 Hz, 2H) ; 3,81 (t, J = 6,0 Hz, 2H) , 7,54 (m, 2H) ; 7,60 (d, J = 5,5 Hz, 1 H) ; 7,78 (m, 1H) ; 7,95 (m large, 1 H) ; 8,34 (d, J = 5,5 Hz, 1H) ; 8,97 (s, 1H) ; 12,95 (m très étalé, 1 H) .
- spectre de masse (E/I) : m/z = 302 (M+)

### Exemple 2 : 1-{1-H-6-hydroxy-benzimidazol-2-yl}-3,4-dihydro-2H-pyrido[2,1-a] isoindole-6-one

*Etape 1:* Dans un tricol de 25 mL, on dissout 942 mg d'ester méthylique de l'acide 3,4-dihydro-2H-pyrido[2,1-a]isoindole-6-one-1-carboxylique, qui peut être obtenu selon Eur. J. Org. Chem. 1999, 3489, dans 10 mL de dioxane et on ajoute 168 mg d'hydroxyde lithium monohydrate. Après 6 heures d'agitation à température ambiante, on évapore le dioxane et on reprend avec 20 mL d'eau, puis on amène à pH=2 par addition d'une solution aqueuse 1M d'acide chlorhydrique. Le précipité formé est essoré, lavé à l'eau puis séché à l'étuve sous vide à 50°C. On obtient ainsi 820 mg d'acide 3,4-dihydro-2H-pyrido[2,1-a] isoindole-6-one-1-carboxylique, sous forme d'une poudre blanche utilisée telle quelle à l'étape suivante.

*Etape 2 :* Dans un tricol de 25 mL, on dissout 442,5 mg d'acide 3,4-dihydro-2H-pyrido[2,1-a]isoindole-6-one-1-carboxylique dans 20 mL de dichlorométhane, puis on ajoute 400 mg de chlorhydrate de 1-(3-diméthylaminopropyl)-3-éthyl-carbodiimide (EDCI) et 270 mg de 1-hydroxybenzotriazole et on agite à température ambiante pendant 1 heure. Après concentration du solvant sous pression réduite, on ajoute successivement 10 mL de tétrahydrofurane et 276 mg de 4-méthoxybenzèn-1,2-diamine et on porte à 70°C pendant 5 heures. On évapore le milieu réactionnel et on obtient ainsi 680 mg du mélange des deux amides régioisomères, utilisé tel quel à l'étape suivante.

*Etape 3:* Dans un tricol de 100 mL, on dissout 118 mg du mélange de régioisomères obtenu précédemment dans 5 mL d'acide trifluoroacétique et 0,5 mL d'anhydride trifluoroacétique et on chauffe à 85°C pendant 2,5 heures. Après concentration sous pression réduite, le résidu est purifié par flash chromatographie sur gel de silice, en éluant avec un mélange d'oxyde de diisopropyle et de méthanol (95/5 en volumes). On obtient ainsi 97 mg de 1-{1-H-6-méthoxy-benzimidazol-2-yl}-3,4-dihydro-2H-pyrido[2,1-a]isoindole-6-one, sous forme d'une poudre beige dont les caractéristiques sont les suivantes :
- spectre de masse (E/I) : m/z = 331 (M+)

*Etape 4 :* Dans un tricol de 25 mL, on agite 20 heures à température ambiante une solution de 61 mg de 1-{1-H-6-méthoxy-benzimidazol-2-yl}-3,4-dihydro-2H-pyrido[2,1-a]isoindole-6-one dans 2 mL d'acide acétique et 3 mL d'une solution aqueuse à 48 % d'acide bromhydrique. Le milieu réactionnel est jeté dans 100 mL d'eau et neutralisé avec une solution saturée d'hydrogénocarbonate de sodium en présence de 25 mL de dichlorométhane. La phase aqueuse est réextraite 2 fois avec 20 mL de dichlorométhane. Les phases organiques jointes sont séchées sur sulfate de magnésium et concentrées sous pression réduite. Le produit brut est obtenu par chromatographie sur gel de silice en éluant avec des mélanges de dichlorométhane et de méthanol (95/5 puis 90/10 en volumes), on obtient ainsi 49 mg de 1-{1-H-6-hydroxy-benzimidazol-2-yl}-3,4-dihydro-2H-pyrido[2,1-a]isoindole-6-one, sous forme d'une meringue blanche dont les caractéristiques sont les suivantes :
- Spectre de masse (E/I) : m/z = 317 (M+).
- Spectre de RMN 1 H (400 MHz DMSO-d6) : 2,04 (m, 2H) ; 2,83 (t, J = 6,0 Hz, 2H) ; 3,80 (t, J = 6,0 Hz, 2H), 6,74 (d large, J = 8,5 Hz, 1H) ; 6,90 (m large, 1H) ; 7,42 (m large, 1H) ; 7,53 (m, 2H) ; 7,75 (m, 1H) ; 8,02 (m large, 1H) ; 9,22 (m étalé, 1 H) ; 12,4 (m très étalé, 1H) .

### Exemple 3: COMPOSITION PHARMACEUTIQUE

On a préparé des comprimés répondant à la formule suivante :
Produit de l'exemple 1 ........................ 0,2 g
Excipient pour un comprimé terminé à..... 1g
(détail de l'excipient : lactose, talc, amidon, stéarate de magnésium).

### Tests biologiques permettant de caractériser biologiquement les produits de l'invention

Le phosphate inorganique libéré au cours de l'hydrolyse de l'ATP par l'activité ATPasique de Hsp82 est quantifié par la méthode du green Malachite. En présence de ce réactif, il y a formation du complexe phosphate inorganique-molybdate-vert de malachite qui absorbe à une longueur d'onde de 620 nm. Les produits à évaluer sont incubés dans un volume réactionnel de 30 µl, en présence de 1µM Hsp82 et de 250 µm de substrat (ATP) dans un tampon composé de 50 mM Hepes-NaOH (pH 7.5), 1 mM DTT, 5 mM MgCl2 et 50 mM KCI à 37°C pendant 60 minutes. Parallèlement, une gamme de phosphate inorganique comprise entre 1 à 40 µM est constituée dans le même tampon. L'activité ATPasique est ensuite révélée par l'addition de 60 µl du réactif biomol green (Tebu). Après 20 minutes d'incubation à température ambiante, l'absorbance des différents puits est mesurée à l'aide d'un lecteur de microplaque à 620 nm. La concentration en phosphate inorganique de chaque échantillon est alors calculée à partir de la courbe d'étalonnage. L'activité ATPasique d'Hsp82 est exprimée en concentration de phosphate inorganique produit en 60 minutes. L'effet des divers produits testés est exprimé en pourcentage d'inhibition de l'activité ATPasique.

La formation d'ADP due à l'activité ATPasique de Hsp82 a été utilisée pour mettre au point une autre méthode d'évaluation de l'activité enzymatique de cette enzyme par application d'un système de couplage enzymatique faisant intervenir la pyruvate kinase (PK) et la lactate déshydrogensase (LDH). Dans cette méthode spectrophotométrique de type cinétique, la PK catalyse la formation d'ATP et de pyruvate à partir de phosphoenol-pyruvate (PEP) et de l'ADP produit par HSP82. Le pyruvate formé, substrat de la LDH, est ensuite transformé en lactate en présence de NADH. Dans ce cas, la diminution de la concentration en NADH, mesurée par la diminution de l'absorbance à la longueur d'onde de 340 nm est proportionnelle à la concentration en ADP produit par HSP82.

Les produits testés sont incubés dans un volume réactionnel de 100 µl de tampon composé de 100 mM Hepes-NaOH (pH 7.5), 5 mM MgCl2, 1 mM DTT, 150 mM KCl, 0.3 mM NADH, 2.5 mM PEP et 250 µM ATP. Ce mélange est préincubé à 37°C pendant 30 minutes avant addition de 3.77 unités de LDH et 3.77 unités de PK. La réaction est initiée par addition du produit à évaluer, en concentrations variables, et de Hsp82, à la concentration de 1 µM. La mesure de l'activité enzymatique de Hsp82 est alors réalisée, en continu, dans un lecteur de microplaque, à 37°C, à la longueur d'onde de 340 nm. La vitesse initiale de la réaction est obtenue par la mesure de la pente de la tangente à l'origine de la courbe enregistrée. L'activité enzymatique est exprimée en µM d'ADP formé par minute. L'effet des divers produits testés est exprimé en pourcentage d'inhibition de l'activité ATPasique selon la codification ci-dessous :
- A : IC50 < 1 µM
- B : 1 µM < IC50 < 10µM
- C : 10µM < IC50 < 100 µM

### Tableau des résultats

| Exemple | Structure | Hsp82 ATPase IC50 µM |
|---|---|---|
| 1 A000187458 | 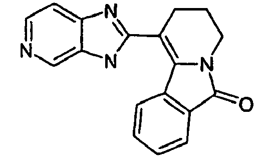 | B |
| 2 A003338065 | 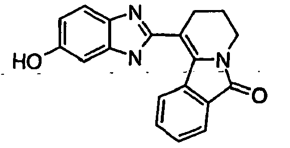 | C |

## Revendications

1. Produits de formule (I) : 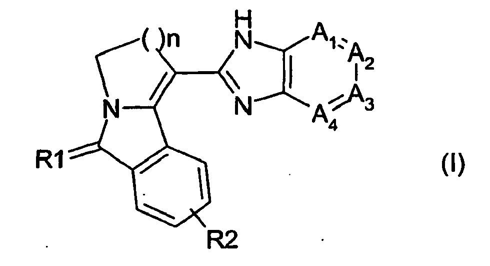 dans laquelle :
A1, A2, A3 et A4, identiques ou différents, représentent CRa ou N ;
n représente l'entier 1 ou 2;
R1 représente un atome d'oxygène ou de soufre ou un radical NRb ;
R2 indépendamment sélectionné dans le groupe constitué par H, halogène, CF3, nitro, cyano, méthyle, éthyle, hydroxy, mercapto, amino, méthoxy, thiométhoxy, méthylamino, diméthylamino, acétylamino, carboxy et carboxamido;
Ra est sélectionné dans le groupe constitué par H, halogène, CF3, hydroxy, mercapto, nitro, amino, OR3, SR3, NR3R4, NH-OH, NH-CO-H, NH-CO-OH, NH-CO-NH2, carboxy, cyano, carboxamido, Y-(CH2)p-alkyle, Y-(CH2)p-cycloalkyle, Y-(CH2)p-hétérocycloalkyle, Y-(CH2)p-aryle ou Y-(CH2)p-hétéroaryle, avec Y = O, S, NH, O-C(O), C(O)-NH, NH-C(O), NH-S(O) ou NH-S(O)2, avec p = 1, 2 ou 3, et dans lequel le radical aryle, contient de 6 à 10 chaînons, le radical cycloalkyle contient de 3 à 10 chaînons et le radical hétéroraryle ou hétérocycloalkyle, contient de 4 à 10 chaînons dont 1 à 3 hétéroatomes choisis parmi O, N, ou S; tous ces radicaux étant éventuellement substitués,
R3 et R4 sont indépendamment choisis parmi l'atome d'hydrogène ou les radicaux alkyles, alkényles, alkynyles, aryles ou hétéroaryle, aralkyle ou hétéroaralkyles ; tous les radicaux alkyles, alkènyles, alkynyles, aryles,
hétéroaryles, aralkyles ou hétéroaralkyles ci-dessus sont éventuellement substitués ;
Rb est sélectionné dans le groupe constitué par H, (CH2)m-cycloalkyle, (CH2)m-hétérocycloalkyle, (CH2)m-aryle ou (CH2)m-hétéroaryle, avec m = 0, 1, 2, tous ces radicaux étant éventuellement substitués,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

2. Produits de formule (I) telle que définie à la revendication 1 dans laquelle A1, A2, A3 et A4, identiques ou différents, sont tels que A1 et A4 identiques ou différents représentent CRa et A2 et A3 représentent N ou CRa,
les substituants n; Ra, R1 et R2 ayant les significations indiquées à l'une quelconque des autres revendications,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

3. Produits de formule (I) telle que définie à l'une quelconque des autres revendications dans laquelle
A1, A2, A3 et A4, identiques ou différents, sont tels que A1 et A4 identiques ou différents représentent CRa et A2 et A3 représentent N ou CRa, avec Ra représente H ou OH,
les substituants n, R1 et R2 ayant les significations indiquées à l'une quelconque des autres revendications,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

4. Produits de formule (I) telle que définie à l'une quelconque des autres revendications dans laquelle
A1, A2, A3 et A4 sont tels que A1 et A4 représentent CH et A2 et A3 identiques ou différents représentent N, CH ou COH,
les substituants n; R1 et R2 ayant les significations indiquées à l'une quelconque des autres revendications,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

5. Produits de formule (I) telle que définie à l'une quelconque des autres revendications dans laquelle R1 représente un atome d'oxygène ou un radical NRb avec Rb représente H ou (CH2)m-hétéroaryle, avec m = 0, 1, 2;
les substituants A1, A2, A3, A4, n et R2 ayant les significations indiquées à l'une quelconque des autres revendications;
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

6. Produits de formule (I) telle que définie à l'une quelconque des autres revendications dans laquelle
n représente 2,
les substituants A1, A2, A3, A4, R1 et R2 ayant les significations indiquées à l'une quelconque des autres revendications,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

7. Produits de formule (I) telle que définie à la revendication 1 dans laquelle A1, A2 et A4 représentent CH et A3 représentent N, CH ou COH,
n représente l'entier 2;
R1 représente un atome d'oxygène;
R2 représente H,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

8. Produits de formule (I) telle que définie à l'une quelconque des revendications précédentes dont les noms suivent :
- le 1-{3-H-imidazo[4,5-c]pyridin-2-yl}-3,4-dihydro-2H-pyrido[2,1-a]isoindole-6-one
- le 1-{1-H-6-hydroxy-benzimidazol-2-yl}-3,4-dihydro-2H-pyrido[2,1-a]isolndole-6-one
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

9. A titre de médicaments, les produits de formule (I) telle que définie aux revendications 1 à 7, , lesdits produits de formule (I) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (I).

10. A titre de médicaments, les produits de formule (I) telle que définie à la revendication 8, lesdits produits de formule (1) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (1).

11. Les compositions pharmaceutiques contenant à titre de principe actif, l'un au moins des médicaments tels que définis aux revendications 9 et 10.

12. Compositions pharmaceutiques selon la revendication 11 **caractérisées en ce qu'**elles sont utilisées comme médicaments, en particulier pour la chimiothérapie de cancers.

13. Compositions pharmaceutiques telles que définies aux revendications précédentes 11 et 12 contenant en plus, des principes actifs d'autres médicaments de chimiothérapie contre le cancer.

14. Utilisation de produits de formule (I) telle que définie à l'une quelconque des revendications précédentes 1 à 8 ou de sels pharmaceutiquement acceptables desdits produits de formule (I) pour la préparation d'un médicament destiné à prévenir ou traiter une maladie **caractérisée par** le dérèglement de l'activité de la protéine HSP90.

15. Utilisation de produits de formule (I) tels que définis à l'une quelconque des revendications précédentes 1 à 8 ou de sels pharmaceutiquement acceptables desdits produits de formule (I) pour la préparation de médicaments destinés à inhiber l'activité de la protéine HSP90.

16. Utilisation de produits de formule (I) selon l'une quelconque des revendications précédentes 1 à 8 dans laquelle la maladie à prévenir ou traiter est chez un mammifère.

17. Utilisation de produits de formule (I) telle que définie à l'une quelconque des revendications précédentes 1 à 8 ou de sels pharmaceutiquement acceptables desdits produits de formule (I) pour la préparation d'un médicament destiné à traiter des cancers.

18. Utilisation de produits de formule (I) selon la revendication précédente 17 dans laquelle la maladie à traiter est un cancer de tumeurs solides ou liquides.

19. Utilisation de produits de formule (I) selon la revendication précédente 18 dans laquelle la maladie à traiter est un cancer résistant aux agents cytotoxiques.

20. Utilisation de produits de formule (I) telle que définie à l'une quelconque des revendications précédentes 1 à 8 ou de sels pharmaceutiquement acceptables desdits produits de formule (I) pour la préparation d'un médicament destiné à traiter des cancers parmi lesquels les cancers du poumon, du sein et de l'ovaire, les glioblastomes, les leucémies myéloïdes chroniques, les leucémies lymphoblastiques aigües, les cancers de la prostate, du pancréas et du colon, les mélanomes métastatiques, les tumeurs de la thyroïde et les carcinomes rénaux.

21. Utilisation de produits de formule (I) telle que définie à l'une quelconque des revendications précédentes 1 à 8 ou de sels pharmaceutiquement acceptables desdits produits de formule (I) pour la préparation de médicaments destinés à la chimiothérapie de cancers utilisés seuls ou en association.

22. Utilisation de produits de formule (I) telle que définie à l'une quelconque des revendications précédentes 1 à 8 ou de sels pharmaceutiquement acceptables desdits produits de formule (I) pour la préparation de médicaments destinés à être utilisés seuls ou en association avec chimiothérapie ou radiothérapie ou alternativement en association avec d'autres agents thérapeutiques.

23. Utilisation de produits de formule (I) selon la revendication précédente 22 dans laquelle les agents thérapeutiques peuvent être des agents anti-tumoraux utilisés communément.

24. Produits de formule (I) tels que définis à l'une quelconque des revendications 1 à 8 comme inhibiteurs de HSP90, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I) .

## Claims

1. Products of formula (I): 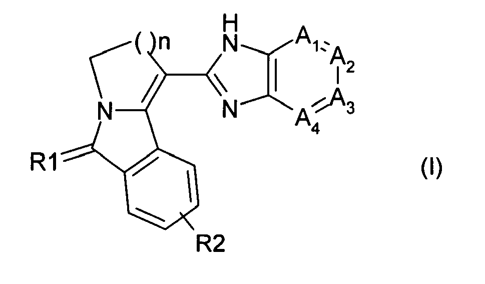 in which:
A1, A2, A3 and A4, which may be identical or different,
are CRa or N ;
n is the integer 1 or 2;
R1 is an oxygen or sulphur atom or a radical NRb;
R2 is independently selected from the group comprising H, halogen, CF3, nitro, cyano, methyl, ethyl, hydroxyl, mercapto, amino, methoxy, thiomethoxy, methylamino, dimethylamino, acetylamino, carboxyl and carboxamido;
Ra is selected from the group comprising H, halogen, CF3, hydroxyl, mercapto, nitro, amino, OR3, SR3, NR3R4, NH-OH, NH-CO-H, NH-CO-OH, NH-CO-NH2, carboxyl, cyano, carboxamido, Y-(CH₂)p-alkyl, Y-(CH₂)p-cycloalkyl, Y-(CH₂)p-heterocycloalkyl, Y-(CH₂)p-aryl or Y-(CH₂)p-heteroaryl, with Y = O, S, NH, O-C(O), C(O)-NH, NH-C(O), NH-S(O) or NH-S(O)2, with p = 1, 2 or 3, and in which the aryl radical contains from 6 to 10 ring members, the cycloalkyl radical contains from 3 to 10 ring members and the heteroaryl or heterocycloalkyl radical contains from 4 to 10 ring members, including 1 to 3 hetero atoms chosen from O, N or S; all these radicals being optionally substituted,
R3 and R4 are independently chosen from a hydrogen atom or alkyl, alkenyl, alkynyl, aryl, heteroaryl, aralkyl or heteroaralkyl radicals; all the alkyl, alkenyl, alkynyl, aryl, heteroaryl, aralkyl or heteroaralkyl radicals above are optionally substituted ;
Rb is selected from the group comprising H, (CH₂)m-cycloalkyl, (CH₂)m-heterocycloalkyl, (CH₂)m-aryl or (CH₂)m-heteroaryl, with m = 0, 1 or 2, all these radicals being optionally substituted,
said products of formula (I) being in all the possible racemic, enantiomeric and diastereoisomeric isomer forms, and also the addition salts with inorganic and organic acids or with inorganic and organic bases of said products of formula (I).

2. Products of formula (I) as defined in claim 1, in which:
A1, A2, A3 and A4, which may be identical or different, are such that A1 and A4, which may be identical or different, are CRa and A2 and A3 are N or CRa,
the substituents n; Ra, R1 and R2 having the meanings indicated in any one of the other claims,
said products of formula (I) being in all the possible racemic, enantiomeric and diastereoisomeric isomer forms, and also the addition salts with inorganic and organic acids or with inorganic and organic bases of said products of formula (I).

3. Products of formula (I) as defined in any one of the other claims, in which:
A1, A2, A3 and A4, which may be identical or different, are such that A1 and A4, which may be identical or different, are CRa and A2 and A3 are N or CRa, with Ra being H or OH,
the substituents n, R1 and R2 having the meanings indicated in any one of the other claims,
said products of formula (I) being in any of the possible racemic, enantiomeric and diastereoisomeric isomer forms, and also the addition salts with inorganic and organic acids or with inorganic and organic bases of said products of formula (I).

4. Products of formula (I) as defined in any one of the other claims, in which:
A1, A2, A3 and A4 are such that A1 and A4 are CH and A2 and A3, which may be identical or different, are N, CH or COH,
the substituents n; R1 and R2 having the meanings indicated in any one of the other claims,
said products of formula (I) being in all the possible racemic, enantiomeric and diastereoisomeric isomer forms, and also the addition salts with inorganic and organic acids or with inorganic and organic bases of said products of formula (I).

5. Products of formula (I) as defined in any one of the other claims, in which:
R1 is an oxygen atom or a radical NRb with Rb being H or (CH₂)m-heteroaryl, with m = 0, 1 or 2 ;
the substituents A1, A2, A3, A4, n and R2 having the meanings indicated in any one of the other claims ;
said products of formula (I) being in all the possible racemic, enantiomeric and diastereoisomeric isomer forms, and also the addition salts with inorganic and organic acids or with inorganic and organic bases of said products of formula (I).

6. Products of formula (I) as defined in any one of the other claims in which:
n is 2,
the substituents A1, A2, A3, A4, R1 and R2 having the meanings indicated in any one of the other claims,
said products of formula (I) being in all the possible racemic, enantiomeric and diastereoisomeric isomer forms, and also the addition salts with inorganic and organic acids or with inorganic and organic bases of said products of formula (I).

7. Products of formula (I) as defined in Claim 1, in which:
A1, A2 and A4 are CH and A3 is N, CH or COH,
n is the integer 2 ;
R1 is an oxygen atom;
R2 is H,
said products of formula (I) being in all the possible racemic, enantiomeric and diastereoisomeric isomer forms, and also the addition salts with inorganic and organic acids or with inorganic and organic bases of said products of formula (I).

8. Products of formula (I) as defined in any one of the preceding claims, having the following names:
- 1-{3-H-imidazo[4,5-c]pyridin-2-yl}-3,4-dihydro-2H-pyrido[2,1-a] isoindole-6-one,
- 1-{1-H-6-hydroxybenzimidazol-2-yl}-3,4-dihydro-2H-pyrido[2,1-a] isoindole-6-one,
said products of formula (I) being in all the possible racemic, enantiomeric and diastereoisomeric isomer forms, and also the addition salts with inorganic and organic acids or with inorganic and organic bases of said products of formula (I).

9. As medicinal products, the products of formula (I) as defined in Claims 1 to 7, said products of formula (I) being in all the possible racemic, enantiomeric and diastereoisomeric isomer forms, and also the pharmaceutically acceptable addition salts with inorganic and organic acids or with inorganic and organic bases of said products of formula (I).

10. As medicinal products, the products of formula (I) as defined in Claim 8, said products of formula (I) being in all the possible racemic, enantiomeric and diastereoisomeric isomer forms, and also the pharmaceutically acceptable addition salts with inorganic and organic acids or with inorganic and organic bases of said products of formula (I).

11. Pharmaceutical compositions containing, as active ingredient, at least one of the medicinal products as defined in Claims 9 and 10.

12. Pharmaceutical compositions according to Claim 11, **characterized in that** they are used as medicinal products, in particular for cancer chemotherapy.

13. Pharmaceutical compositions as defined in the preceding Claims 11 and 12, also containing active ingredients of other medicinal products for cancer chemotherapy.

14. Use of products of formula (I) as defined in any one of the preceding Claims 1 to 8 or of pharmaceutically acceptable salts of said products of formula (I), for preparing a medicinal product for use in preventing or treating a disease **characterized by** the disturbance of the activity of the Hsp90 protein.

15. Use of products of formula (I) as defined in any one of the preceding Claims 1 to 8 or of pharmaceutically acceptable salts of said products of formula (I), for preparing medicinal products for use in inhibiting the activity of the Hsp90 protein.

16. Use of products of formula (I) according to any one of the preceding Claims 1 to 8, in which the disease to be prevented or treated is in a mammal.

17. Use of products of formula (I) as defined in any one of the preceding Claims 1 to 8 or of pharmaceutically acceptable salts of said products of formula (I), for preparing a medicinal product for use in treating cancers.

18. Use of products of formula (I) according to the preceding Claim 17, in which the disease to be treated is a cancer consisting of solid or liquid tumours.

19. Use of products of formula (I) according to the preceding Claim 18, in which the disease to be treated is a cancer resistant to cytotoxic agents.

20. Use of products of formula (I) as defined in any one of the preceding Claims 1 to 8 or of pharmaceutically acceptable salts of said products of formula (I), for preparing a medicinal product for use in treating cancers, among which are lung cancer, breast cancer and ovarian cancer, glioblastomas, chronic myeloid leukaemias, acute lymphoblastic leukaemias, prostate cancer, pancreatic cancer and colon cancer, metastatic melanomas, thyroid tumours and renal carcinomas.

21. Use of products of formula (I) as defined in any one of the preceding Claims 1 to 8 or of pharmaceutically acceptable salts of said products of formula (I), for preparing medicinal products for use in cancer chemotherapy, used alone or in combination.

22. Use of products of formula (I) as defined in any one of the preceding Claims 1 to 8 or of pharmaceutically acceptable salts of said products of formula (I), for preparing medicinal products to be used alone or in combination with chemotherapy or radiotherapy, or alternatively in combination with other therapeutic agents.

23. Use of products of formula (I) according to the preceding Claim 22, in which the therapeutic agents can be commonly used antitumour agents.

24. Products of formula (I) as defined in any one of Claims 1 to 8, as Hsp90 inhibitors, said products of formula (I) being in all the possible racemic, enantiomeric and diastereoisomeric isomer forms, and also the pharmaceutically acceptable addition salts with inorganic and organic acids or with inorganic and organic bases of said products of formula (I).

## Patentansprüche

1. Produkte der Formel (I): 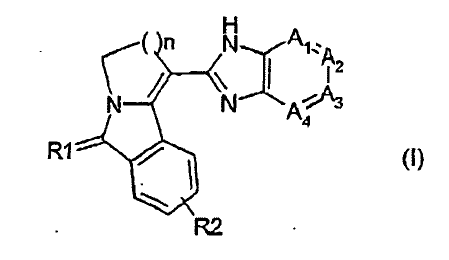 worin:
A1, A2, A3 und A4 gleich oder verschieden sind und für CRa oder N stehen;
n für die ganze Zahl 1 oder 2 steht;
R1 für ein Sauerstoff- oder Schwefelatom oder einen Rest NRb steht;
R2 unabhängig aus der Gruppe bestehend aus H, Halogen, CF3, Nitro, Cyano, Methyl, Ethyl, Hydroxyl, Mercapto, Amino, Methoxy, Thiomethoxy, Methylamino, Dimethylamino, Acetylamino, Carboxyl und Carboxamido ausgewählt ist;
Ra aus der Gruppe bestehend aus H, Halogen, CF3, Hydroxyl, Mercapto, Nitro, Amino, OR3, SR3, NR3R4, NH-OH, NH-CO-H, NH-CO-OH, NH-CO-NH2, Carboxyl, Cyano, Carboxamido, Y-(CH₂)p-Alkyl, Y-(CH₂)p-Cycloalkyl, Y-(CH₂)p-Heterocycloalkyl, Y-(CH₂)p-Aryl oder Y-(CH₂)p-Heteroaryl mit Y = O, S, NH, O-C(O), C(O)-NH, NH-C(O), NH-S(O) oder NH-S(O)2 und mit p = 1, 2 or 3 ausgewählt ist, wobei der Arylrest 6 bis 10 Glieder enthält, der Cycloalkylrest 3 bis 10 Glieder enthält und der Heteroaryl- oder Heterocycloalkylrest 4 bis 10 Glieder enthält, von denen 1 bis 3 Heteroatome sind, die unter O, N oder S ausgewählt sind und alle diese Reste gegebenenfalls substituiert sind;
R3 und R4 unabhängig unter einem Wasserstoffatom oder Alkyl-, Alkenyl-, Alkinyl-, Aryl-, Heteroaryl-, Aralkyl- oder Heteroaralkylresten ausgewählt sind, wobei alle obigen Alkyl-, Alkenyl-, Alkinyl-, Aryl-, Heteroaryl-, Aralkyl-oder Heteroaralkylreste gegebenenfalls substituiert sind;
Rb aus der Gruppe bestehend aus H, (CH₂)m-Cycloalkyl, (CH₂)m-Heterocycloalkyl, (CH₂)m-Aryl oder (CH₂)m-Heteroaryl mit m = 0, 1 oder 2 ausgewählt ist, wobei alle diese Reste gegebenenfalls substituiert sind;
wobei die Produkte der Formel (I) in allen möglichen racemischen, enantiomeren und diastereomeren isomeren Formen vorliegen, sowie die Additionssalze der Produkte der Formel (I) mit anorganischen und organischen Säuren oder mit anorganischen und organischen Basen.

2. Produkte der Formel (I) nach den Ansprüchen 1, worin
A1, A2, A3 und A4 gleich oder verschieden sind und so beschaffen sind, daß A1 und A4 gleich oder verschieden sind und für CRa stehen und A2 und A3 für N oder CRa stehen,
wobei die Substituenten n, Ra, R1 und R2 die in einem der anderen Ansprüche angegebene Bedeutung besitzen,
wobei die Produkte der Formel (I) in allen möglichen racemischen, enantiomeren und diastereomeren isomeren Formen vorliegen, sowie die Additionssalze der Produkte der Formel (I) mit anorganischen und organischen Säuren oder mit anorganischen und organischen Basen.

3. Produkte der Formel (I) nach einem der anderen Ansprüche, worin
A1, A2, A3 und A4 gleich oder verschieden sind und so beschaffen sind, daß A1 und A4 gleich oder verschieden sind und für CRa stehen und A2 und A3 für N oder CRa stehen, wobei Ra für H oder OH steht,
wobei die Substituenten n, R1 und R2 die in einem der anderen Ansprüche angegebene Bedeutung besitzen,
wobei die Produkte der Formel (I) in allen möglichen racemischen, enantiomeren und diastereomeren isomeren Formen vorliegen, sowie die Additionssalze der Produkte der Formel (I) mit anorganischen und organischen Säuren oder mit anorganischen und organischen Basen.

4. Produkte der Formel (I) nach einem der anderen Ansprüche, worin
A1, A2, A3 und A4 so beschaffen sind, daß A1 und A4 für CH stehen und A2 und A3 gleich oder verschieden sind und für N, CH oder COH stehen, wobei die Substituenten n, R1 und R2 die in einem der anderen Ansprüche angegebene Bedeutung besitzen,
wobei die Produkte der Formel (I) in allen möglichen racemischen, enantiomeren und diastereomeren isomeren Formen vorliegen, sowie die Additionssalze der Produkte der Formel (I) mit anorganischen und organischen Säuren oder mit anorganischen und organischen Basen.

5. Produkte der Formel (I) nach einem der anderen Ansprüche, worin R1 für ein Sauerstoffatom oder einen Rest NRb steht, worin Rb für H oder (CH₂)m-Heteroaryl mit m = 0, 1 oder 2 steht,
die Substituenten A1, A2, A3, A4, n und R2 die in einem der anderen Ansprüche angegebene Bedeutung besitzen,
wobei die Produkte der Formel (I) in allen möglichen racemischen, enantiomeren und diastereomeren isomeren Formen vorliegen, sowie die Additionssalze der Produkte der Formel (I) mit anorganischen und organischen Säuren oder mit anorganischen und organischen Basen.

6. Produkte der Formel (I) nach einem der anderen Ansprüche, worin
n für 2 steht,
die Substituenten A1, A2, A3, A4, R1 und R2 die in einem der anderen Ansprüche angegebene Bedeutung besitzen,
wobei die Produkte der Formel (I) in allen möglichen racemischen, enantiomeren und diastereomeren isomeren Formen vorliegen, sowie die Additionssalze der Produkte der Formel (I) mit anorganischen und organischen Säuren oder mit anorganischen und organischen Basen.

7. Produkte der Formel (I) nach Anspruch 1, worin
A1, A2 und A4 für CH stehen und A3 für N, CH oder COH steht,
n für die ganze Zahl 2 steht,
R1 für ein Sauerstoffatom steht,
R2 für H steht,
wobei die Produkte der Formel (I) in allen möglichen racemischen, enantiomeren und diastereomeren isomeren Formen vorliegen, sowie die Additionssalze der Produkte der Formel (I) mit anorganischen und organischen Säuren oder mit anorganischen und organischen Basen.

8. Produkte der Formel (I) nach einem der anderen Ansprüche mit den folgenden Namen:
- 1-{3-H-Imidazo[4,5-c]pyridin-2-yl}-3,4-dihydro-2H-pyrido[2,1-a]isoindol-6-on,
- 1-{1-H-6-Hydroxybenzimidazol-2-yl}-3,4-dihydro-2H-pyrido[2,1-a]isoindol-6-on,
wobei die Produkte der Formel (I) in allen möglichen racemischen, enantiomeren und diastereomeren isomeren Formen vorliegen, sowie die Additionssalze der Produkte der Formel (I) mit anorganischen und organischen Säuren oder mit anorganischen und organischen Basen.

9. Produkte der Formel (I) nach den Ansprüchen 1 bis 7, wobei die Produkte der Formel (I) in allen möglichen racemischen, enantiomeren und diastereomeren isomeren Formen vorliegen, sowie die pharmazeutisch unbedenklichen Additionssalze der Produkte der Formel (I) mit anorganischen und organischen Säuren oder mit anorganischen und organischen Basen als Medikament.

10. Produkte der Formel (I) nach Anspruch 8, wobei die Produkte der Formel (I) in allen möglichen racemischen, enantiomeren und diastereomeren isomeren Formen vorliegen, sowie die pharmazeutisch unbedenklichen Additionssalze der Produkte der Formel (I) mit anorganischen und organischen Säuren oder mit anorganischen und organischen Basen als Medikament.

11. Pharmazeutische Zusammensetzungen, die als Wirkstoff mindestens eines der Medikamente nach den Ansprüchen 9 und 10 enthalten.

12. Pharmazeutische Zusammensetzungen nach Anspruch 11, **dadurch gekennzeichnet, daß** sie als Medikamente verwendet werden, insbesondere für die Krebschemotherapie.

13. Pharmazeutische Zusammensetzungen nach den vorhergehenden Ansprüchen 11 und 12, die außerdem Wirkstoffe anderer Medikamente für die Krebschemotherapie enthalten.

14. Verwendung von Produkten der Formel (I) nach einem der vorhergehenden Ansprüche 1 bis 8 oder von pharmazeutisch unbedenklichen Salzen der Produkte der Formel (I) zur Herstellung eines Medikaments zur Prävention oder Behandlung einer Erkrankung, die durch die Störung der Aktivität des HSP90-Proteins **gekennzeichnet** ist.

15. Verwendung von Produkten der Formel (I) nach einem der vorhergehenden Ansprüche 1 bis 8 oder von pharmazeutisch unbedenklichen Salzen der Produkte der Formel (I) zur Herstellung von Medikamenten zur Inhibierung der Aktivität des HSP90-Proteins.

16. Verwendung von Produkten der Formel (I) nach einem der vorhergehenden Ansprüche 1 bis 8, wobei die zu verhindernde oder zu behandelnde Erkrankung bei einem Säugetier auftritt.

17. Verwendung von Produkten der Formel (I) nach einem der vorhergehenden Ansprüche 1 bis 8 oder von pharmazeutisch unbedenklichen Salzen der Produkte der Formel (I) zur Herstellung eines Medikaments zur Behandlung von Krebserkrankungen.

18. Verwendung von Produkten der Formel (I) nach dem vorhergehenden Anspruch 17, wobei es sich bei der zu behandelnden Erkrankung um Krebs mit soliden oder flüssigen Tumoren handelt.

19. Verwendung von Produkten der Formel (I) nach dem vorhergehenden Anspruch 18, wobei es sich bei der zu behandelnden Erkrankung um einen Krebs handelt, der gegenüber Cytotoxika resistent ist.

20. Verwendung von Produkten der Formel (I) nach einem der vorhergehenden Ansprüche 1 bis 8 oder von pharmazeutisch unbedenklichen Salzen der Produkte der Formel (I) zur Herstellung von Medikamenten zur Behandlung von Krebserkrankungen, u.a. Lungen-, Brust- und Eierstockkrebs, Glioblastomen, chronischen myeloischen Leukämien, akuten lymphoblastischen Leukämien, Prostatakrebs, Bauchspeicheldrüsenkrebs und Kolonkrebs, metastatischen Melanomen, Schilddrüsentumoren und Nierenkarzinomen.

21. Verwendung von Produkten der Formel (I) nach einem der vorhergehenden Ansprüche 1 bis 8 oder von pharmazeutisch unbedenklichen Salzen der Produkte der Formel (I) zur Herstellung von Medikamenten für die Krebschemotherapie, die allein oder in Kombination verwendet werden.

22. Verwendung von Produkten der Formel (I) nach einem der vorhergehenden Ansprüche 1 bis 8 oder von pharmazeutisch unbedenklichen Salzen der Produkte der Formel (I) zur Herstellung von Medikamenten zur alleinigen Verwendung oder zur Verwendung in Kombination mit Chemotherapie oder Strahlentherapie oder alternativ dazu in Kombination mit anderen Therapeutika.

23. Verwendung von Produkten der Formel (I) nach dem vorhergehenden Anspruch 22, wobei es sich bei den Therapeutika um gängige Antitumormittel handeln kann.

24. Produkte der Formel (I) nach einem der Ansprüche 1 bis 8, wobei die Produkte der Formel (I) in allen möglichen racemischen, enantiomeren und diastereomeren isomeren Formen vorliegen, sowie die pharmazeutisch unbedenklichen Additionssalze der Produkte der Formel (I) mit anorganischen und organischen Säuren oder mit anorganischen und organischen Basen als Inhibitoren von HSP90.
